# EUROPEAN PATENT APPLICATION

(11) **EP 1 688 621 A2**
(43) Date of publication of application: **09.08.2006**
(21) Application number: 05016572.9
(22) Date of filing: 29.07.2005
(51) Int. Cl.: F04C 29/00, F04C 18/356

(54) **Hand-held vacuum pump and automated urinary drainage system using that vacuum pump**

(30) Priority: 20.01.2005 JP 2005012223; 14.02.2005 JP 2005035371
(71) Applicant: Hitachi, Ltd., Tokyo 100-8280 (JP)
(72) Inventor: Tazoe, Wataru, Tsuchiura-shi Ibaraki 300-0022 (JP); Miyagawa, Ryosuke, Kasukabe-shi Saitama 344-0032 (JP); Ishitsuka, Yoshikazu, Ibaraki 319-0104 (JP); Tanaka, Tetsuya, Tokyo 134-0083 (JP); Azuhata, Kiyoshi, Ishioka-shi Ibaraki 315-0035 (JP); Koromegawa, Isao, Tsuchiura-shi Ibaraki 300-0841 (JP)
(74) Representative: Beetz & Partner

(57) **Abstract**

The invention relates to a hand-held or portable vacuum pump and a urine drainage system comprising that vacuum pump. In the hand-held vacuum pump (1), the main body (1a) of the vacuum pump in which the pump room (4a) is formed and the electric motor (2) are connected via a shaft coupling (21). The main body (1a) of the vacuum pump (1) has a driving shaft (10). One ball bearing (15b) supporting the driving shaft (10) is supported by the cylinder (4). The other ball bearing (15a) is supported by a flange (5) mounted hermetically at the cylinder (4). The end part of the cylinder at the side of the electric motor (2) accommodates the shaft coupling (21), and is engaged with the end part of the electric motor (2) so that the electric motor (2) may support the cylinder (4).

## Description

### BACKGROUND OF THE INVENTION

The present invention relates to a hand-held vacuum pump, and specifically to a hand-held vacuum pump preferable for an automated urinary drainage system.

An example of the urine suction apparatus for urinary drainage for the people finding difficulty in moving on his or her own ability to the place where he or she relieve himself or herself is described in the Japanese Patent Application Laid-Open No. 2003-126242. In the urine suction apparatus sited in this Patent Gazette, a urine storage container is located between the urine receiver and the suction pump operated as the suction engine. When the urine is discharged into the urine receiver, the urine discharge is detected and the discharged urine is sucked from the suction pipeline into the urine storage container. In this operation, the suction operation of the suction pump is controlled by referring to the signal from the pressure sensor for detecting the changes in the pressure inside the urine storage container.

A lightweight vacuum pump is used generally for the suction pump. An example of this type of vacuum pump is described in the Japanese Patent Application Laid-Open No. 2002-195180 and the Japanese Patent Application Laid-Open No. 8-296575. In the compressor described in the Japanese Patent Application Laid-Open No. 2002-195180, in order to improve the sliding characteristic and ultimately the efficiency, such a sliding member as providing a continuous lubrication function by means of self-lubricating operation is provided at the rotary compressor. In contrast, in the movable vane compressor and vacuum pump described in the Japanese Patent Application Laid-Open No. 8-296575, the compressor room and the pump room for the sealing fluid are partitioned separately and hermetically, and the sealing fluid discharged out from the pump room is supplied to the sealing part and the lubricating part.

In the conventional urine suction apparatus sited in the Japanese Patent Application Laid-Open No. 2003-126242, what is disclosed is that the operation state of the suction pump operated as suction engine is changed in response to the pressure inside the urine storage container. However, in the Japanese Patent Application Laid-Open No. 2003-126242, there is not sufficient consideration for downsizing the suction pump and reducing its operation noise. In case of downsizing the overall system in order to increase the portability of the urine suction apparatus, it is required to downsize the suction pump itself. Although one of the most effective methods for downsizing the pump is to increase the rotating speed of the pump, in case of attempting to increase the rotating speed of the pump, especially the positive displacement pump generally used as the small-sized pump, the relative gap between the rotating member and the stationary member increases which leads to reducing the efficiency. In addition, increasing the rotating speed simply may result in the increase in the noise generated from the pump. As for the urine suction apparatus, the usage out of doors as well as the usage bedside especially in the night should be considered, which requires inevitably the reduction of the noise.

In contrast, in the compressor sited in the Japanese Patent Application Laid-Open No. 2003-126242 and Japanese Patent Application Laid-Open No. 8-296575, the reliability of the sealing part and the lubricating part is increased in order to prevent the reduction in efficiency due to the downsizing of the overall system. However, as this compressor is not intended to be used for the hand-held medical device such as urinary drainage system, the downsizing of the compressor may experience a complexity in the structure of the compressor itself that is the result of the design respecting the performance primarily. In addition, the intended use of the compressor does not assume the usage in the night, and thus, there is not sufficient consideration of the reduction of the noise generated in the intermittent operation of the compressor.

### BRIEF SUMMARY OF THE INVENTION

In order to solve the problems in the prior art, an object of the present invention is to downsize the vacuum pump and increase its portability. Another object of the present invention is to increase the reliability of the vacuum pump having portability. Further another object of the present invention is to enable the urinary drainage system having a vacuum pump to be used in the night and out of doors.

The characterized feature of the present invention to achieve the above object is that, in the hand-held vacuum pump in which the electric motor and the main body of the vacuum pump are connected through the shaft coupling, one bearing supporting the driving shaft included in the main body of the vacuum pump is supported by the cylinder and the other bearing is supported by the flange mounted hermetically at the cylinder, and the end part of the cylinder near the electric motor accommodates the shaft coupling and is engaged with the end part of the electric motor, and the electric motor supports the cylinder.

In the characterized feature described above, the round hole to be engaged with the rotating shaft of the electric motor is formed at one side of the shaft coupling and the quasi-square hole to be engaged with the driving shaft passing through inside the cylinder is formed at the other side of the shaft coupling, and the hole to be engaged with the rotating shaft of the electric motor is formed at the driving shaft, which may eliminates preferably the locking screw for fixing the shaft coupling and the rotating shaft of the electric motor. Inaddition, it is preferable that the material of the shaft coupling is made to be re sin, and plural protruding parts extending in the axial direction on the inside face of the round hole formed at the shaft coupling are formed, or that plural holes extending in the radial direction communicating with this round hole are formed.

In the characterized feature described above, it is preferable that a couple of sliding cylinders having a circular arc part and clamping both sides of the intermediate part of the extension part configured as a plate structure is provided, and this sliding cylinder is supported by the cylinder and the sliding cylinder is made oscillating inside the cylinder, and the cylinder and the piston are formed as double cylinders, and that the pump room is formed so that the shape of the pump room may change between the double cylinders.

In the characterized feature described above, it is preferable that the piston is mounted at the driving shaft through the eccentric shaft, this piston has a blade part configured as a plate structure extending towards the outer side at a part of its periphery, the sliding cylinder having a circular arc part supports this blade part so as to freely oscillate, and that the material of the driving shaft is made to be stainless steel, the material of the shaft coupling is made to be PC resin or POM resin, the material of the piston is made to be PS resin or PC resin, the material of the cylinder is made to be PBT resin, and the material of the sliding cylinder is made to be PPS resin or PBT resin. Note that PC stands for Polycarbonade, POM stands for Polyacetal, PS stands for Polystyrene, PPS stands for Polyphenylene Sulfide, and PBT stands for Polybutylene Terphthalate.

In addition, in the characterized feature described above, it is preferable that the balance weight is mounted at the end part of the driving shaft for compensating the eccentricity between the piston and the eccentric shaft, and the cover for covering the balance weight is mounted at the flange, and the material of the flange is made to be POM resin, and the cylinder and the piston are formed as double cylinders, and that the gas inside the pump room is compressed by changing the volume of the pump room formed between the double cylinders by oscillating the blade part.

In addition, in the characterized feature described above, it is preferable that the beveled cut part having a cross-sectional shape of shaft shaped in D-sigmoid is formed at the driving shaft, and the hole corresponding to this D-sigmoid shape is formed at the eccentric shaft, and plural protruding parts extending in the axial direction are formed inside the hole shaped in D-sigmoid formed at the eccentric shaft, and that the support part supporting the sliding cylinder is formed at the cylinder, and the suction passage for sucking the gas into the cylinder and the discharge passage for discharging the gas are mounted near this support part and the double cylinder part so as to be almost vertical to the vibrating blade part, and a valve configured in a sheet structure for opening and closing the discharge passage and a discharge valve presser for pressing down this valve are provided at the discharge passage. In addition, it is preferable that the diameter of the hand -held vacuum pump is 30mm or shorter, its length is 70 mm or shorter, and its weight is 250g or lighter, and the electric motor is driven by the dry cell or the secondary battery providing an output voltage between 4V and 9V, and that the overall exhaust velocity when driven by this vacuum pump at 4000 to 6000 rpm is about 80mL/sec.

In another characterized feature of the present invention, the automated urinary drainage system includes the hand-held vacuum pump having either one of the characterized aspects described above which provides the exhaust speed around 80 mL/sec and the weight of 250g or lighter.

According to the present invention, as the structure of the vacuum pump is simplified, it will be appreciated that the downsizing of the vacuum can be attained and the portability can be increased. As the positioning accuracy at the individual parts is increased by using a simplified structure and the lubrication between the stationary parts and the moving parts are sufficiently provided, it will be appreciated that the reliability of the vacuum pump having portability can be increased. As the positioning accuracy is increased, it will be appreciated that the number of noise generation sources and their noise intensity can be reduced, and the urinary drainage system to which this vacuum pump is applied can be used in the night and/or out of doors.

### BRIEF DESCRIPTION OF THE SEVERAL VIEWS OF THE DRAWINGS

FIG. 1 is a side cross-section view of one embodiment of the hand-held vacuum pump of the present invention.
FIG. 2 is an A-A cross-section view of FIG. 1.
FIG. 3 is a perspective exploded view of the rotor part of the vacuum pump shown in FIG. 1.
FIG. 4 is a perspective exploded view of the shaft coupling of the vacuum pump shown in FIG. 1.
FIG. 5 is a perspective exploded view of the driving shaft part used for the vacuum pump shown in FIG. 1.
FIG. 6 is a side view of the shaft coupling used for the vacuum pump shown in FIG. 1.
FIG. 7 is a perspective exploded view of another embodiment of the shaft coupling used for the vacuum pump shown in FIG. 1.
FIG. 8 is a side view of the end part of the driving shaft used for the vacuum pump shown in FIG. 1.
FIG. 9 is a front view and a side view of the discharge valve stopper 19 used for the vacuum pump shown in FIG. 1.

### DETAILED DESCRIPTION OF THE INVENTION

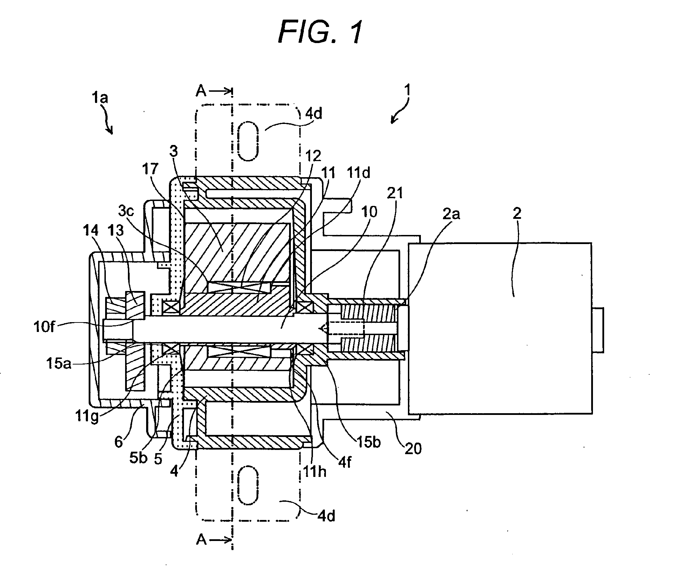

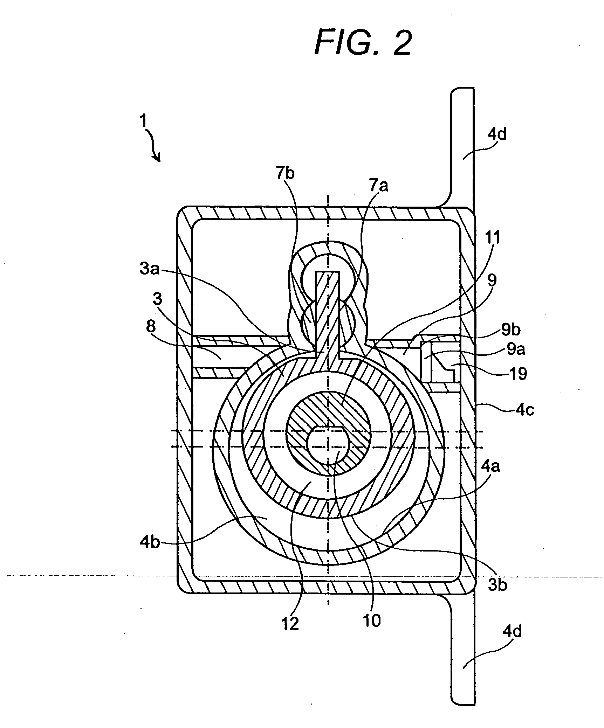

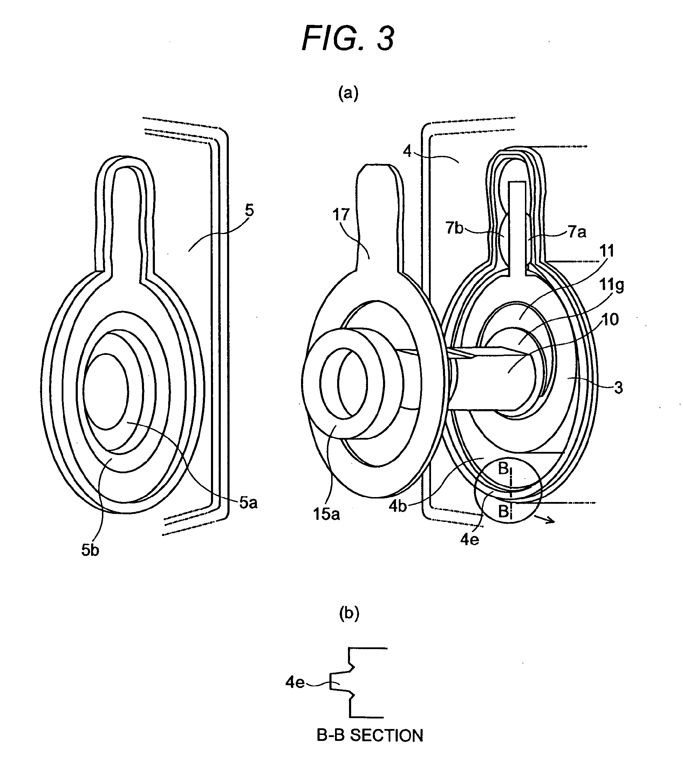

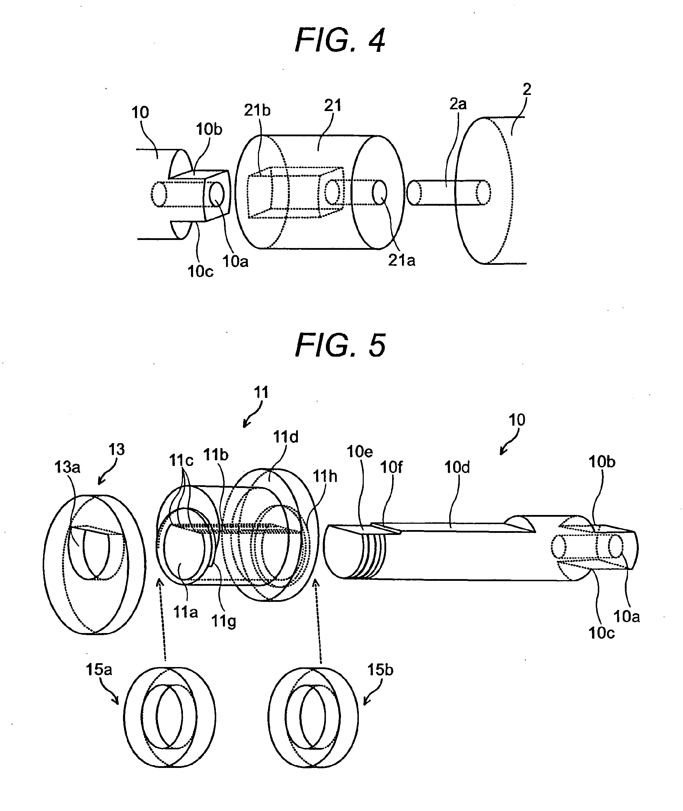

Now referring to the attached figures, one embodiment of the hand -held vacuum pump according to the present invention will be described. The vacuum pump in this embodiment is mainly used for the urinary drainage system. FIG. 1 is a vertical cross section view of the vacuum pump, and FIG. 2 is an A-A cross-section view of FIG. 1. FIG. 3 is an exploded and perspective view of the main part of the vacuum pump shown in FIG. 1 and its B-B cross-section view, and FIG. 4 is an exploded and perspective view of the end part of the shaft of the vacuum pump shown in FIG. 1, and FIG. 5 is an exploded and perspective view of the shaft of the vacuum pump shown in FIG. 1.

In the hand-held vacuum pump 1, the main body 1 a of the vacuum pump is connected to one side of the driving motor 2 through the shaft coupling 21. The motor 2 drives the vacuum pump 1 at the rotating speed between 4000rpm and 7000rmp. The diameter of the motor 2 is about 30mm, and its axial length is about 35mm. The motor 2 is driven by the dry cell or the secondary battery providing the voltage between 4V and 9V.

The intermediate part in the axial direction of the driving shaft 10 connected to the motor shaft 2a through the shaft coupling 21 is engaged to the hole formed at the eccentric position inside the cylindrical eccentric shaft 11, and the eccentric shaft 11 is driven around the eccentric axis around the motor shaft 2a. The needle bearing 12 is engaged at the periphery of the eccentric shaft 11. Both right and left ends in the axial direction of the eccentric shaft 11 are supported by the ball bearings 15a and 15b so as to rotate freely. The balance weight 13 is mounted at the contact face 10f of the eccentric shaft 11 at the opposite side of the motor, that is, the left end part shown in FIG. 1, on the basis of the contact face of the balance weight. The balance weight 13 is supported at the eccentric shaft 11 by the stopper 14.

In order to prevent the lubricant which is coated at the ball bearing 15a supported at the ball bearing supporting part 5a shaped in a cylinder, but is not shown in the figure, from flying in all directions, the lubricant reservoir dish 5b shaped in a cone is mounted around the ball bearing supporting part 5a. In case of installing the needle bearing 12 between the piston 3 and the eccentric shaft 11, the needle bearing fixing part 11 d for supporting the needle bearing 12 is formed by pressing the inner ring of the needle bearing 12 in the axial direction.

The part of the driving shaft 10 on the side of the balance weight 13 is covered by the cover 6 engaged with the flange 5. The fixing plate 4d for fixing the vacuum pump 1 at the urinary drainage system is provided on one side 4c of the cylinder 4. In addition, the end part of the cylinder 4 on the side of the motor 2 is shaped in a cylinder, and accommodates the shaft coupling 21 and is engaged with the engage part formed at the electric motor 2. Owing to this structure, the cylinder 4 can be directly supported by the motor 2. The outer part of the cylinder corresponding to the pump room 4a is formed as a double structure, and the motor base 20 for supporting the periphery of the motor 2 is mounted at the end part of this double structure on the side of motor 2. Thus, the cylinder 4 is supported by the motor 2 also with the motor base 20.

The ball bearing 15b is supported at the cylinder 4 integrated with the casing which is a container shaped like a "Snowman" and has an open structure at the left side shown in FIG. 1. On the other hand, the left side ball bearing 15a is supported by the ball bearing supporting part 5a formed at the flange 5. The flange 5 is a member configured at the in a plate structure covering the open face of the cylinder 4, and the dedicated sheet 17 is arranged between the flange and the cylinder so as to be engaged hermetically with the cylinder 4. The dedicated sheet 17 is a sheet composed of PTFE, and used for compensating the molding error and the assembling error of the cylinder 4 composed of a resin material, the eccentric shaft 11, the piston 3 and the sliding cylinders 7a and 7b, which will be described in detail below.

As the cylinder 4 slides on the dedicated sheet 17, the dedicated sheet is composed of easy-to-slide material. The dedicated sheet 17 can change its shape when sliding with the cylinder 4. The piston 3 is mounted at the periphery of the needle bearing 12, and this piston 3 have a ring and a blade 3a formed by extending one part on the circumferential direction of this ring toward the outer side. The eccentric shaft 11, the needle bearing 12 and the piston 3 are accommodated inside the cylinder 4.

The intermediate part of the blade 3a is clamped at the left and right sides by the sliding cylinders 7a and 7b forming a part of the cylinder. The sliding cylinders 7a and 7b are supported by the protruding part of the cylinder 4 with its inner surface shaped so as to be fit to the external shape of the sliding cylinders 7a and 7b. The head part of the blade 3a extends towards the space shaped in a elliptic cylinder formed at the inner face of the protruding part of the cylinder 4. The lower part of the piston 3 is shaped in a cylinder, and its peripheral surface 3b faces to the inner surface 4a of the lower part of the cylinder 4 shaped in a cylinder. The gap between the piston 3 and the cylinder 4 establishes a very little clearance in order to keep the sealing performance even at the narrowest gap. The piston 3 has a swirling motion inside the cylinder 4. When the piston 3 swirls with a constant radius, the bicephalic space 4b formed between the inner surface 4a of the cylinder 4 and the piston 3 moves by following to this swirling motion. Then, the gaseous component is taken in from the intake passage 8 and discharged to the discharge passage 9.

The intake passage 8 and the discharge passage 9 are formed in the vertical direction orthogonal to the driving shaft 4 near the supporting part of the cylinder 4 for supporting the sliding cylinders 7a and 7b. The intake passage 8 and the discharge passage 9 are open to the pump room 4b. The valve seat 9b is formed at the discharge passage 9, and the discharge valve 9a contacts to this valve seat 9b. The discharge valve 9a is a silicon sheet having a thickness of 0.5mm. The discharge valve stopper 19 presses down the discharge valve 9a towards the opposite direction of the discharge flow. The discharge valve 9a to be mounted at the discharge passage 9 is mounted at the location that is possibly located on the internal surface 4a of the cylinder and near the blade 3a in order to reduce the dead volume. In this embodiment, the valve seat 9b is formed by forming a hole larger than the diameter of the discharge passage 9 on the side of cylinder side face at the discharge passage.

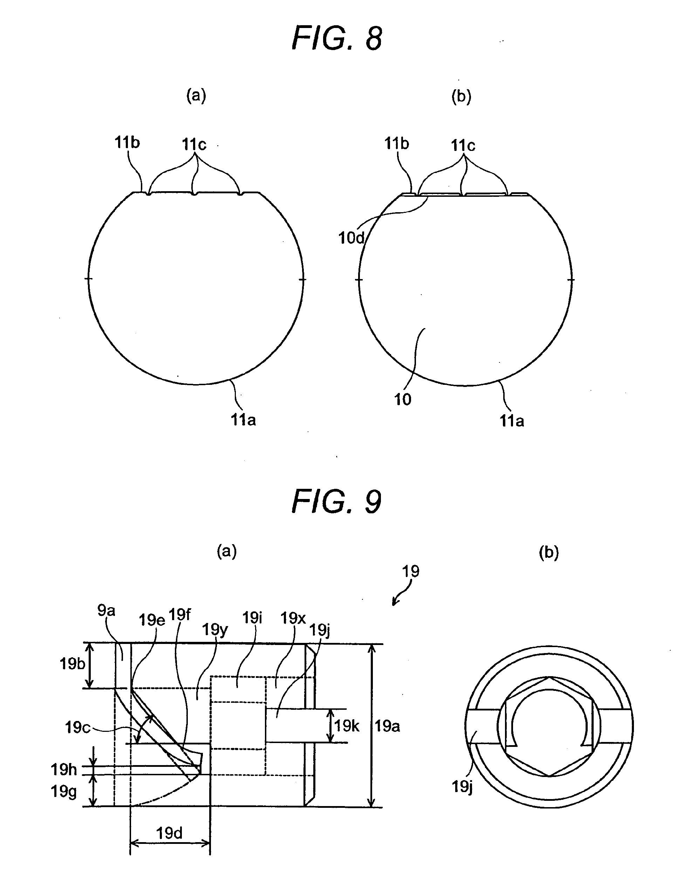

The detail structure of the discharge valve stopper 19 is shown in FIG. 9. FIG. 9 (a) shows the front view, and FIG. 9 (b) shows the side view. The discharge valve stopper 19 is formed by processing and forming a round -bar material. A through -hole 19y is formed at th e center part of the round bar, and a stepped shape having the step size 19d equivalent to the discharge valve stroke in the axial direction is formed at its back face. As shown in FIG. 9, a part of the step has a canted part. The slotted part 19j, which is a groove developed in the radial direction, is formed at the front side of the round bar, and the round hole 19x larger than the diameter of the through hole 19y is formed up to the intermediate part in the axial direction. The hexagon hole 19i is formed at the backside of the round hole 19x, which is used for mounting the discharge vale stopper 19 by using the hexagonal wrench. The diameter 19a of the discharge valve stopper 19 is 5.0mm.

The discharge valve 9a having the diameter almost identical to the diameter of the discharge valve stopper 19 is arranged at the backside of the discharge valve stopper 19. The discharge valve 9a is a circular plate made of PTFE with 0.1mm thickness, and its top part to the stepped part are pressed down by the discharge valve stopper 19 in accordance with the shape of the discharge valve stopper 19. Thus, the discharge valve can bend right and left at its stepped part to its lower part due to the pressure inside the discharge passage 9. The valve stopper length 19b, measured from the top part to the stepped part, is 1.15mm. The valve aperture angle 19c, which is the canted angle of the stepped part, is 45 degrees. The discharge valve 9a bends with the supporting points at the valve aperture supporting parts 19e and 19f, which are located at the both ends of the canted parts. As the lower end part of the discharge valve 9a can bend with the supporting point at the valve aperture supporting part 19f, the communication channel between the front side of the discharge valve stopper 19 and the backside of the discharge valve 9a can be established when the discharge valve 9a bends.

As the height of the air discharge port 19h is established the inner side of the thick part 19g of the ring formed by the penetration hole 19y formed at the discharge valve stopper 19, the channel communicating from the penetration hole 19 y to the slotted part 19j of the groove width 19K is formed. Owing to this structure, the discharge valve can be operated definitely independently of the installation state of the vacuum pump. Note that the noise generated by opening and closing the discharge valve 9a can be reduced because the stepped part is formed as a canted shape.

As shown in detail in FIG. 4, the driving shaft 10 and the rotating shaft 2a of the motor 2 are linked together with the shaft coupling 21. The shaft coupling 21 is shaped in a cylinder, and has a round hole 21 a formed at the side where the rotating shaft 2a of the motor 2 is engaged with the shaft coupling. On the other hand, a quasi-rectangular hole (square hole) 21 b which is formed by cutting a circle with a couple of lines is formed at the side where the driving shaft 10 is engaged with the shaft coupling. A fillet having a couple of planes 10b and 10c parallel to each other is formed at the driving shaft 10 corresponding to this square hole. A round hole 10a with which the rotating shaft of the motor can be engaged is formed at the center part of the driving shaft 10.

In the vacuum pump 1 so configured as described above, the flange 5 and the dedicated sheet 17 are hermetically mounted at the cylinder 4, and then the pump room 4b is formed. The protruding part 4e is formed on the surface of the cylinder facing to the flange 5, and this protruding part is transformed when installing the flange 5 at the cylinder 4, and thus, the protruding part 4e and the dedicated sheet 17 firmly contact to the flange 5. Owing to this structure, the sealing performance of the pump room 4b is established.

When the motor shaft 2a rotates, the eccentric shaft 11 rotates in an eccentric motion, and then the piston 3 mounted at the peripheral side of the eccentric shaft also rotates in an eccentric motion. In this operation, the movement of the blade 3a of the piston 3 is limited by the sliding cylinders 7a and 7b, and then the rotation of the piston 3 on its own axis is blocked. The material and shape (as a part of the cylinder) of the sliding cylinders 7a and 7b blocking the rotation of the blade 3a on its own axis is selected to be appropriate for sliding performance in order to make the movement of the blade 3a smooth.

In this embodiment, the rotation driving power of the motor 2 is transmitted to the driving shaft 10 by using the static friction generated between the rotating shaft 2a of the motor and the round hole 21a. When the shaft coupling 21 rotates, the turning force is transmitted from the square hole 21 b formed at the shaft coupling 21 to the flat surfaces 10b and 10c of the fillet of the driving shaft 10, and thus the driving shaft 10 rotates. Though the shaft coupling 21 was fixed at the shaft 10 by using the clamp screw in the prior art, this embodiment does not use the clam screw. The reason is that the application of the clamp screw makes the weight of the clamp screw itself operate as unbalanced load that may result in an occurrence of vibration. As this embodiment does not use the clamp screw, it will be appreciated that the driving shaft 10 and the shaft coupling 21 can be engaged firmly. In addition, in view of the disassembly operation for the driving shaft 10 and the shaft coupling 21, PC (Polycarbonate) resin is used for the material of the shaft coupling 21, which is aimed for weight saving and abrasion resistance.

Though this embodiment uses the shaft coupling 21 made of Polycarbonate for the reason described above, it is difficult for the shaft coupling 21 made of the resin material to establish the same level of molding accuracy as the metallic shaft coupling. In order to prevent the axial alignment deviation between the rotating shaft 2a of the motor 2 and the driving shaft 10 due to the molding error, this embodiment uses such a structure that the rotating shaft 2a of the motor 2 can be inserted into the round hole 10a. Owing to this structure, the vibration at the high-speed rotation which may result from the slight deviation in the axial alignment between the rotating shaft 2a of the motor 2 and the driving shaft 10 can be reduced as well as the mechanical loss can be prevented. In addition, the axial length of the vacuum pump 1 can be reduced, which contributes to the downsizing of the vacuum pump 1.

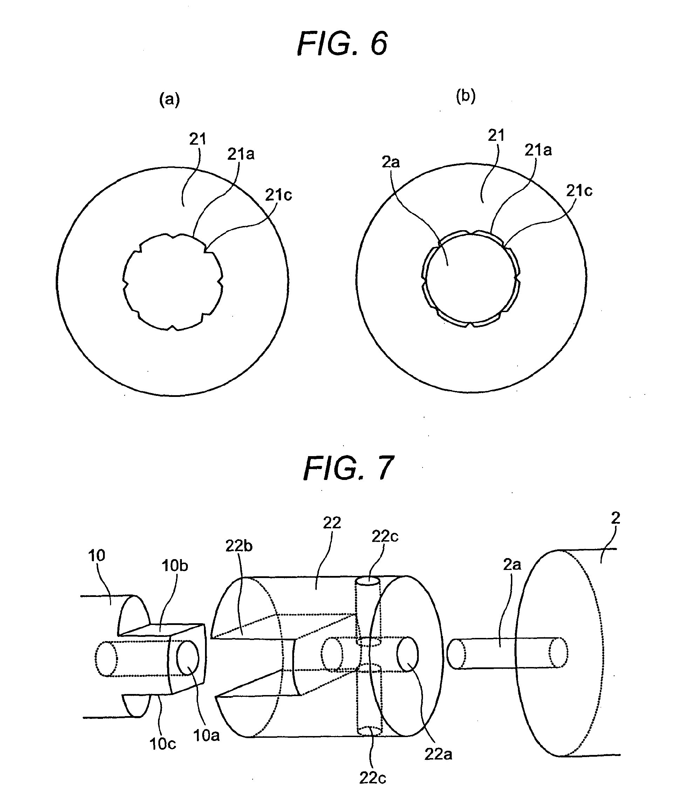

This embodiment uses such a structure that the shaft coupling 21 is not fixed at the rotating shaft 2a of the motor 2 by using the clamp screw and can be dismounted from the motor 2 without using the clamp screw. In the structure of this embodiment, the protruding part 21 c extending in the axial direction shown in detail in FIG. 6 is formed on the inner surface of the round hole 21 a of the shaft coupling 21. Plural protruding parts 21c are formed with an interval in the circumferential direction on the inner surface of the shaft coupling 21 c. FIG. 6 (a) shows the side view of the shaft coupling 21c alone, and FIG. 6 (b) is a cross-section view of the structure in which the shaft coupling 21 is mounted at the rotating shaft 2a of the motor 2. As the rotating shaft 2a is inserted into the shaft coupling 21, the protruding parts 21 c of the shaft coupling 21 made of the resin material are deformed elastically.

As plural protruding parts 21 c are formed on the inner surface of the shaft coupling 21, the rotation torque generated by the rotating shaft 2a of the motor 2 can be transmitted definitely to the shaft coupling 21 owing to the friction force. In addition, owing to the deformation of the protruding parts 21 c, the accuracy of positioning, that is, axial alignment of the rotating shaft 2a of the motor 2 can be established. Owing to the deformation of the protruding parts 21 c, the touch area between the rotating shaft 2a of the motor 2 and the shaft coupling required for transmitting the torque can be ensured. The height of the individual protruding part 21 c is determined so that the gap between the protruding part 21 c and the rotating shaft 2a engaged to each other may not be developed when inserting the rotating shaft 2a of the motor 2. Thus, even if the finished size of the round hole 21 a is a little consistently different from the designed value or its finishing accuracy has a little deviation, and even if the difference between the material of the rotating shaft 2a and the material of the shaft coupling 21 may result in the difference in their heat deformation, there is no gap between the protruding parts 21c and the rotating shaft 2a, and hence, the rotation torque can be definitely transmitted.

The rotation torque is transmitted from the side surface of the square hole 21 b of the shaft coupling 21 and the flat surfaces 10b and 10c of the fillet of the driving shaft 10 to the driving shaft 10. The square hole 21 b and the fillet are used not only for transmitting the torque but also for positioning the parts when assembling the parts. By aligning the flat surfaces 10b and 10c of the fillet onto the two side surfaces of the square hole 21 b, the shaft coupling 21 can be positioned to the driving shaft 10 in the vertical and horizontal directions, respectively. In addition, the rotating shaft 2a of the motor 2 can be positioned to the round hole 10a of the driving shaft 10 only by inserting the fillet into the square hole 21 b of the shaft coupling 21.

FIG. 7 shows another embodiment of the shaft coupling. In the above embodiment, the shaft coupling 21 is fixed at the rotating shaft 2a and the driving shaft 10a by the protruding part 21 c extending in the axial direction and the fillet. In this embodiment, plural rigidity reducing holes 22c extending in the radial direction of the shaft coupling 22 are formed approximately at the axially symmetrical positions. As the shaft coupling 22 is composed of resin material, the inner surface of the rigidity reducing hole 22c is deformed plastically to the inward side when processing the rigidity reducing hole 22c. Due to this deformation, when inserting the rotating shaft 2a into the round hole 22a, the inner surface of the round hole 22a is flared by the rotating shaft 2a and the firm engagement between the rotating shaft 2a and the shaft coupling 22 is established in the circumferential direction without gap. On the side of the driving shaft 10, the groove 22b is formed so as to extend to the periphery corresponding to the shape of the end part of the driving shaft 10. According to this way of forming the shaft coupling 22, the positioning between the motor shaft 2a and the driving shaft 10 can be easily established and the thermal deformation is treated in the similar way to the above embodiment.

Now, referring to FIG. 3 and FIG. 5, the operation of the pump is described. At the one position on the periphery of the driving shaft 10, the slotted surface 10d is formed and the cross-section of the driving shaft is shaped in a circle truncated with a straight line. As the hole (hole for the driving shaft) 11a having the same cross-sectional shape as the cross-section of the driving shaft at the slotted surface 10d is formed at the eccentric shaft 11, the position of the eccentric shaft 11 in the peripheral direction can be determined only by inserting the driving shaft 10 into the hole 11 a for the driving shaft. When the driving shaft 10 rotates, the rotation torque is transmitted from the slotted surface 10d to the flat face 11 b of the hole 11 a for the driving shaft, and then the eccentric shaft 11 rotates. In this operation, the axle center of the eccentric shaft 11 and the axle center of the driving shaft 10 are not aligned to each other, the eccentric shaft 11 rotates in an eccentric motion.

In the above operation, due to the assembly error and gap between the eccentric shaft 11 and the driving shaft 10, the vibration gives rise to the eccentric shaft 11 and the vibration amplitude may increase due to the eccentric rotating motion of the eccentric shaft 11. As a result, the mechanical efficiency is reduced and the friction loss and abrasion at the sliding surface between the cylinder 4 and the piston 3 occur. Consequently, the abrasion and the thermal deformation cause the decrease in the lifetime of the vacuum pump 1. In order to prevent those disadvantages, this embodiment uses the eccentric shaft 11 made of PPS (Polyphenylene Sulfide). By means of using the eccentric shaft 11 made of PPS, as this material is lightweight and advantageous for abrasion resistance and heat resistance as well as relatively low abrasion resistance among other resin materials, the vacuum pump 1 can be operated over the long term.

As the eccentric shaft 11 is lightweight, the unbalanced force distribution (centrifugal force) due to the eccentric rotating motion can be reduced and thus the required mass of the balance weight 13 can be reduced. Owing to this configuration, the downsizing of the vacuum pump can be achieved. Though this embodiment uses such a structure that the driving shaft 10 and the eccentric shaft 11 are engaged to each other, it is allowed to apply the insert molding process to integrate both parts. In case of insert molding, the driving shaft is positioned inside the die assembly and then the resin material for forming the eccentric shaft is filled in this die assembly. By means of forming the eccentric shaft 11 by the insert molding process, the alignment between the eccentric shaft and the driving shaft 10 can be established, which leads to increasing the performance of the compressor and reducing the man-hour cost for fabrication.

In contrast to the resin material used for the eccentric shaft, metallic material which has an advantageous property in mechanical strength is used for the driving shaft 10. As the driving shaft 10 is made of metallic material, the coefficient of thermal expansion of the driving shaft is different from the coefficient of the thermal expansion of the resin-made eccentric shaft 11. Due to the difference in their coefficients of the thermal expansion, the eccentric shaft 11 tends to transform its shape in the peripheral direction to the driving shaft 10. In order to this problem, plural protruding parts 11c is formed so as to extend in the axial direction on the flat face 11b of the eccentric shaft 11. FIG. 8 shows the outline of the hole 11a for the driving shaft formed at the eccentric s haft 11. FIG. 8 (a) is an outline of the hole 11a for the driving shaft alone, and FIG. 8 (b) is a cross-section view when inserting the driving shaft 10 into the hole 11 a.

In the similar manner to the case of the relation between the rotating shaft 2a of the motor 2 and the shaft coupling 21, when the driving shaft 10 is inserted into the hole 11a for the driving shaft, the protruding part 11c extending in the axial direction is deformed elastically or plastically, and then, the gap between the driving shaft 10 and the eccentric shaft 11 goes out of existence. Then, even if the driving shaft 10 and the eccentric shaft 11 yield to the heat deformation due to the heat generated by the rotational movement of the driving shaft 10, the steady state that the gap between he driving shaft 10 and the eccentric shaft 11 does not exist can be maintained owing to the deformation of the protruding part 11 b. Thus, the relative displacement of the eccentric shaft 11 in the circumferential direction relative to the driving shaft 10 can be prevented.

In this embodiment, three protruding parts 11 c extending in the axial direction are formed on the flat face 11 b with its cross-section shaped in D-sigmoid. In addition, as the length measured in the axial direction of the flat face 10d of the driving shaft 10 ad the flat face 11 b of the eccentric shaft 10 is long, the flat faces 10d and 11 b are made slanted in the axial direction in view of easiness for assembly and disassembly. The inclination angle in this slanted structure is about 1 degree. As the vibration due to the existence of the gap between the driving shaft 10 and the eccentric shaft 11 is reduced as in the above-described way, the required mass for the balance weight 13 can be reduced.

A case study of applying the above-described small-sized vacuum pump 1 to the automated urinary drainage system will be described below. The entire component including the vacuum pump 1 is required to be replaced periodically in view of sanitary supervision for the automated urinary drainage system. In order to make the replacement parts recyclable, the component of the vacuum pump 1 is so configured as to be enabled to be disassembled material by material. Table 1 summarizes the analysis of easiness of assembly and disassembly and the preferable materials to be selected.

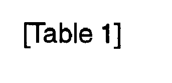

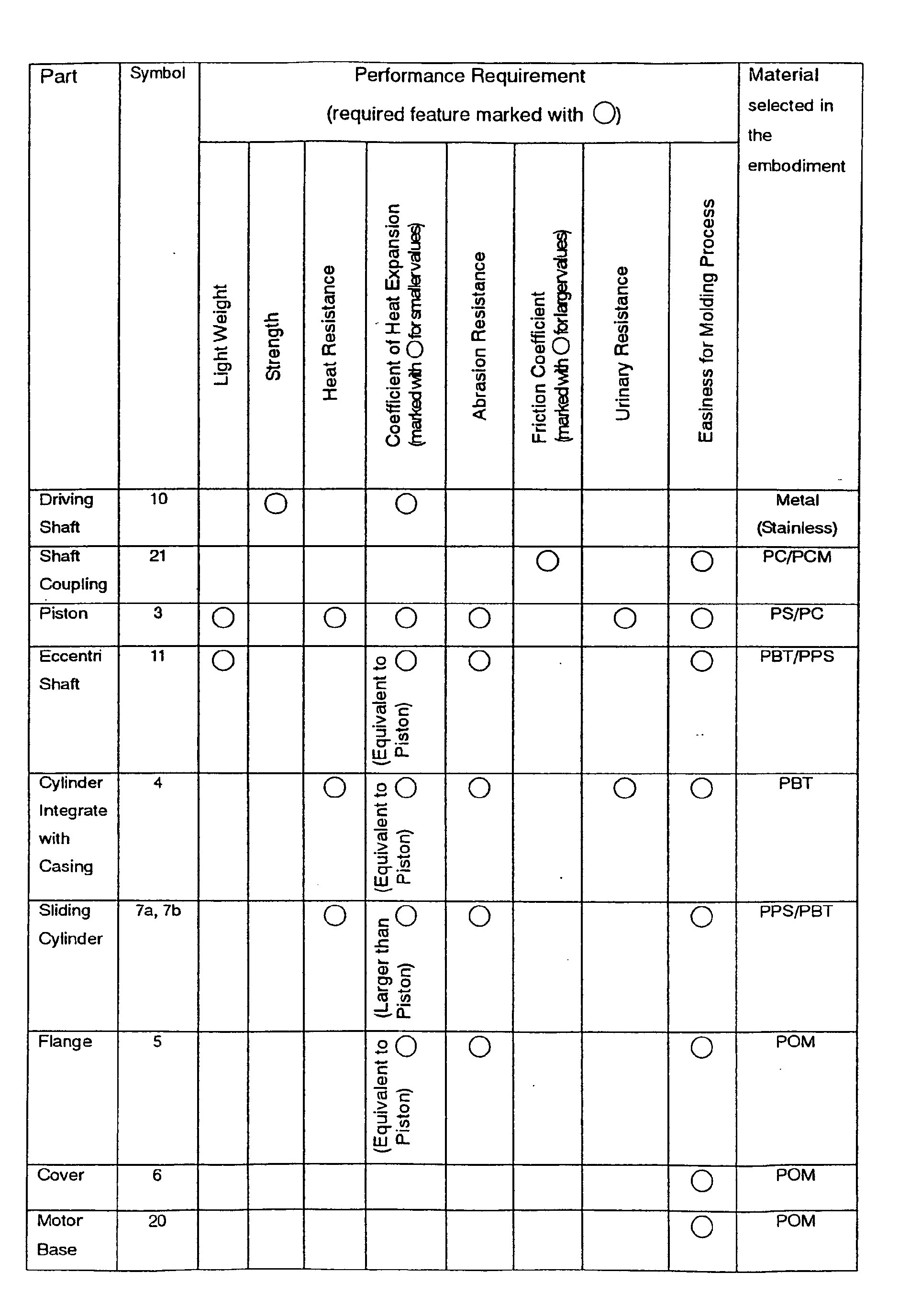

As determined from the above reason, the materials used for the driving shaft 10 and the eccentric shaft 11 are selected to be stainless steel and PBT/PPS, respectively. The material used for the piston 3 is selected to be PC/PC which has such advantageous chemical-resistant properties as weak-acid resistance and weak-alkali resistance. Those materials have an advantageous mechanical deformation property.

The individual parts of the vacuum pump 1 expand due to heat in the operation of the vacuum pump 1. The materials used for the cylinder 4, the eccentric shaft 11 and the sliding cylinders 7a and 7b for guiding the blade part 3a of the piston 3 are made approximately identical to one another in order to reduce the stress generated due to the thermal expansion. In this embodiment, the material used for those parts is determined to PS (Polystyrene). In case of increasing the sliding performance by installing the needle bearing 12 between the eccentric shaft 11 and the driving shaft 10, the needle bearing supporting part 3c for supporting the end face of the needle bearing 12 is formed at the eccentric shaft 11.

Corresponding to the position where the ball bearing 15a and 15b are supported by the cylinder 4, the ball bearing positioning seats 11 g and 11 h for pressing down in the axial direction to the inner face of the ball bearing 15a and 15b are formed at the eccentric shaft 11. Those ball bearing positioning seats 11 g and 11 h are shaped in a cylinder, and their length in the axial direction is about 0.5mm. The size of the eccentric shaft 11 in the axial direction is almost the same as the width of the piston 3 and the pump room 4b.

As for the material to be used for the cylinder, such a material that has an advantageous property in abrasion resistance and heat resistance and has weak-acid resistance and weak-alkali resistance and has a mechanical pro perty of easiness for molding should be selected. As the cylinder 4 slides with the piston 3, the material to be used for the cylinder 4 is selected to be PPS (Polyphenylene Sulfide) or PBT (Polybutylene Terphthalate) having approximately the same coefficient of thermal expansion as the material of the piston 3. The material to be used for sliding cylinders 7a and 7b guiding the blade part 3a of the piston 3 is selected to be the material having an advantageous property in abrasion resistance and heat resistance and having approximately the same coefficient of thermal expansion as the material of the piston 3. In this embodiment, also in view of easiness for molding process, PPS or PBT is used for the material of the sliding cylinders 7a and 7b.

The material to be used for the flange 5 is selected to be the material having easiness for molding process, and having approximately the same coefficient of thermal expansion as the material of the piston 3 and the cylinder 4. The material to be used for the flange requires a certain level of abrasion resistance which might not be less than the material to be used for the other sliding parts. In this embodiment, the material to be used for the flange is Homopolymer of POM (Polyacetal). In view of easiness for molding process, the material to be used for the motor base 2 and cover 6 is selected to be Copolymer of POM.

The size of the vacuum pump is determined on the basis of the drained urinary volume. By referring to the article in Acta Urologica Japonica, Vol. 33, No. 4, pp. 521-526, (April, 1987), which describes the investigation results of the urinary volumes for the individual generations (urinary volume per unit time), the drained urinary volume of the adult people is estimated. According to this p aper, the maximum drained urinary volume of the young adult people between 19 to 39 years old is 28.2 ± 4.6mL per second, which is larger than the volume of any other generation. The maximum drained urinary volume is determined to be 40mL per second on the safer side. By considering the involvement of air and the other fluid dynamics loss, the overall drainage speed of the vacuum pump is determined to be 70mL per second. Thus, assuming that the diameter of the cylinder is 17.2mm, its length is 16.3mm and the diameter of the piston is 14.0mm, the drained volume per single revolution of the piston is approximately 1.066mL, and the urine can be drained by 76.4mL per second by the vacuum pump 1 driven at 4300rpm. Assuming that there exists approximately 10% loss, the urine can be drained approximately by 70mL per minute.

Though the sizes of the individual parts of the vacuum pump and its rotating speed are determined as described above in this embodiment, it is allowed to select another sizes and rotating speed alternatively if the overall drain speed of 70mL per second is attained. As the downsizing and weight saving of the vacuum pump can be established so far, the outline of the vacuum pump system integrated with the motor can be determined so that the diameter may be 30mm or shorter and its length may be 70mm or shorter. Owing to this configuration, it will be appreciated that the dry cell and the secondary battery, including solar cell and fuel cell, having the output voltage approximately from 4V to 9V can be supplied as the electric power unit.

Although the present invention has been illustrated and described with respect to exemplary embodiment thereof, it should be understood by those skilled in the art that the foregoing and various other changes, omission and additions may be made therein and thereto, without departing from the spirit and scope of the present invention. Therefore, the present invention should not be understood as limited to the specific embodiment set out above but to include all possible embodiments which can be embodied within a scope encompassed and equivalent thereof with respect to the feature set out in the appended claims.

## Claims

1. Hand-held vacuum pump (1) comprising an electric driving motor (2) and a pump, the main body (1 a) of the vacuum pump being formed with a pump room (46) and being connected to the motor (2), via a shaft coupling (21), wherein
one bearing (15b) supporting the driving shaft (10) of the main body (1 a) of the vacuum pump is supported by a cylinder (4), and the other bearing (15a) is supported by a flange (5) mounted hermetically at the cylinder (4),
the end part of the cylinder (4) at the side of the electric motor (2) accommodates the shaft coupling (21) and is engaged with the end part of the electric motor (2), and the
electric motor (2) supports the cylinder (4).

2. Hand-held vacuum pump according to claim 1, wherein
a round hole engaged with the rotating shaft (2a) of the electric motor (2) is formed at one side of the shaft coupling (21),
a quasi-square hole engaged with the drive shaft penetrating inside the cylinder (4) is formed at the other side of the shaft coupling (21), and
a hole engaged with the rotating shaft (2a) of the electric motor (2) is formed at the driving shaft (10) so that a locking screw for the shaft coupling (21) and the rotating shaft (2a) of the electric motor (2) may not be required.

3. Hand-held vacuum pump according to claim 1 or 2, wherein
an eccentric shaft made of resin material aligned with an eccentric axis to the driving shaft (10) and having an outline cylindrical part is provided;
a piston (3) having an extension part configured as a plate structure at a part of periphery is mounted at the outer side of said eccentric shaft; and
plural protruding parts extending in the axial direction of the inner face of the eccentric shaft are formed at an engaged part between the driving shaft (10) and the eccentric shaft.

4. Hand-held vacuum pump according to any of claims 1 to 3, wherein
the material to be used for the shaft coupling (21) is selected to be a resin material, and
plural protruding parts extending in the axial direction at the inner face of a round hole formed said shaft coupling, or plural protruding parts extending in a radial direction and connecting to the round hole.

5. Hand-held vacuum pump, optionally according to any of claims 1 to 4, wherein
a couple of sliding cylinders clamping both sides of an intermediate part of an extension part of said plate structure and having a circular arc part is provided,
the sliding cylinder is supported by a cylinder, and the siding cylinder is made oscillating inside the cylinder,
the cylinder and the piston are formed as double cylinders, and
a pump room (4b) is formed so that the shape of the pump room (4b) may change between said double cylinders.

6. Hand-held vacuum pump according to any of claims 1 to 5, wherein
a piston (3) is mounted at the driving shaft through an eccentric shaft,
the piston has a blade part configured as a plate structure extending towards the outer side at a part of the periphery,
the sliding cylinder having a circular arc part supports the blade part so as to freely oscillate,
the material of the driving shaft (10) being stainless steel, the material of the shaft coupling (21) being PC resin or POM resin, the material of the piston (3) being PS resin or PC resin, the material of the cylinder (3) being PBT resin, and the material of the sliding cylinder being PPS resin or PBT resin, wherein PC stands for Polycarbonate, POM stands for Polyacetal, PS stands for polystyrene, PPS stands for polyphenylene sulfide, and PBT stands for polybutylene terphthalate.

7. Hand-held vacuum pump according to any of claims 1 to 6, wherein
a balance weight (13) is mounted at an end part of the driving shaft (10) for compensating the eccentricity between the piston (3) and the eccentric shaft,
a cover for covering the balance weight (13) is mounted at the flange (5), and
the material of the flange (5) is POM resin.

8. Hand-held vacuum pump according to any of claims 1 to 7, wherein
the cylinder and the piston are formed as double cylinders, and
the gas inside the pump room (4b) is compressed by changing the volume of the pump room (4b) formed between the double cylinders by oscillating the blade part.

9. Hand-held vacuum pump according to any of claims 1 to 8, wherein
a beveled cut part having the cross-sectional shape of the shaft shaped in D-sigmoid is formed at the driving shaft,
a hole corresponding to the D-sigmoid shape is formed at the eccentric shaft, and
plural protruding parts extending in an axial direction inside the hole shaped in D-sigmoid are formed at the eccentric shaft.

10. Hand-held vacuum pump according to any of claims 1 to 9, wherein
a support part supporting the sliding cylinder is formed at the cylinder,
a suction passage for sucking a gas into the cylinder and a discharge passage for discharging the gas are mounted near the support part and a double cylinder part so as to be almost vertical to a vibrating blade part,
a valve configured in a sheet structure for opening and closing the discharge passage and a discharge valve presser for pressing down the valve are provided at the discharge passage.

11. Hand-held vacuum pump according to any of claims 1 to 10, wherein
the diameter of the hand-held vacuum pump is 30 mm or shorter, its length is 70 mm or shorter, and its weight is 250 g or lighter,
the electric motor (2) is driven by a dry cell or a secondary battery providing an output voltage between 4 V and 9 V, and
the overall exhaust velocity when driven by the vacuum pump at 4000 to 6000 rpm is about 80 ml/s.

12. Automated urinary drainage system, comprising a hand-held vacuum pump according to any of claims 1 to 11.

13. Automated urinary drainage system according to claims 12, wherein the exhaust rate of the vacuum pump is 70 ml/s or larger, and the weight of the vacuum pump is 250 g or lighter.
